(19) Europäisches Patentamt
European Patent Office
Office européen des brevets

(11) **EP 1 744 161 A1**

(12) **EUROPEAN PATENT APPLICATION**

(43) Date of publication:
**17.01.2007 Bulletin 2007/03**

(51) Int Cl.:
*G01N 33/542* (2006.01)  *G01N 33/543* (2006.01)

(21) Application number: **06253574.5**

(22) Date of filing: **07.07.2006**

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br>Designated Extension States:<br>**AL BA HR MK YU** | (72) Inventors:<br>• **Chamberlin, Danielle**<br>  **Loveland, CO 80537-0599 (US)**<br>• **Roitman, Daniel**<br>  **Loveland, CO 80537-0599 (US)** |
| (30) Priority: **11.07.2005  US 179430** | (74) Representative: **Tollett, Ian et al**<br>**Williams Powell**<br>**Morley House** |
| (71) Applicant: **Agilent Technologies, Inc.**<br>**Palo Alto, CA 94306 (US)** | **26-30 Holborn Viaduct**<br>**London EC1A 2BP (GB)** |

(54) **Substrate for detecting analyte**

(57)    The present invention is directed to a substrate (22) for use in detection of an analyte where the substrate includes an engineered surface (32) having nanostructures (36) and the analyte is labeled with nanoparticles. The substrate also includes at least one molecule (38) having binding affinity for the nano-particle labeled analyte; wherein association of at least one nano-particle labeled analyte to at least one molecule on the engineered substrate causes a detectable change in resonant wavelength or intensity. The present invention also includes a kit comprising the above substrate and methods for use thereof.

FIG. 3

EP 1 744 161 A1

## Description

The present invention is directed to a substrate for use in detection of an analyte, wherein the substrate includes at least one engineered surface. The substrate also includes at least one molecule having binding affinity for a nano-particle labeled analyte, wherein the molecule is associated with the engineered surface. The engineered surface includes nanostructures or high index materials such that association of at least one nano-particle labeled analyte to at least one molecule on the engineered surface causes a detectable signal. The present invention also includes a kit comprising the above substrate and methods for use thereof.

While metal nanoparticles are frequently used as labels in array-based applications, it is well recognized in the art, that traditional applications using gold nanoparticles suffer from lack of visibility (poor detection) due to low signal to noise ratios. As such, there is a need in the art to develop an effective way to substantially improve signal to noise ratios for detection of gold and other nanoparticle labeled molecules in techniques, such as Point of Care Testing (POCT).

In a first aspect of the invention there is provided a substrate as claimed in claim 1.

In a second aspect of the invention there is provided a kit as claimed in claim 6.

In a third aspect of the present invention there is provided a method as claimed in claim 10.

A number of preferred embodiments of the present invention will now be described with reference to the accompanying drawings, in which:-

    Figure 1 is a sectional view of an embodiment of a nanoparticle array.
    Figure 2 is a sectional view of an additional embodiment of a nanoparticle array.
    Figure 3 is a sectional view of an embodiment of a substrate including two nanoparticle arrays.
    Figure 4 is a sectional view of an embodiment of a substrate including an engineered surface with bound nanoparticle-labeled analyte.
    Figure 5 is a sectional view of an additional embodiment of a substrate including an engineered surface with bound nanoparticle-labeled analyte.
    Figure 6 shows a cross-sectional view of a POCT substrate.

Various embodiments of the present invention will be described in detail with reference to the drawings, wherein like reference numerals represent like parts throughout the several views. Reference to various embodiments does not limit the scope of the invention, which is limited only by the scope of the claims attached hereto. Additionally, any examples set forth in this specification are not intended to be limiting and merely set forth some of the many possible embodiments for the claimed invention.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

## Substrate with Engineered Surface

In an embodiment of the present invention, a substrate with an engineered surface is combined with a nano-particle-labeled sample (e.g., analyte), wherein the interaction of the engineered surface and nanoparticle label improves optical detection by shifting detected wavelength, increasing intensity or quenching of an emission peak. The engineered surface changes the environment of the labeling nanoparticles to improve detection (e.g., sensitivity of measurement). In an embodiment, a substrate includes an engineered surface for use in optical measurement. In an embodiment, a shift in wavelength upon nanoparticle-labeled analyte binding to the engineered surface provides a basis for detection.

In an embodiment, a substrate is any solid object having a surface suitable for supporting at least one engineered surface. In an embodiment, substrates include, but are not limited to: strips, dipsticks, slides, wafers, paper, cups, cells, wells, and plates. In an embodiment, a substrate includes one engineered surface. In another embodiment, a substrate includes two or more engineered surfaces. In an embodiment, a substrate is suitable for use in POCT.

In an embodiment, the present invention relates to a kit, substrate and method for detection of at least one analyte in point of care testing (POCT) of a sample. Point-of-care testing (POCT) is testing that is designed to be employed at the point of care, such as in emergency rooms, operating rooms, hospital laboratories and other clinical laboratories, doctor's offices, in the field, or in any situation in which a rapid and accurate result is desired. POCT systems, devices and methods process data diagnostic tests or assays, including immunoassays, chemical assays, nucleic acid assays, calorimetric assays, fluorometric assays, chemiluminescent and bioluminescent assays, and other such tests, and provide an indication of a medical condition or risk or absence thereof.

The engineered surface includes a plurality of nanostructures immobilized in a region on a surface of a substrate. In an embodiment, the engineered surface also comprises immobilized molecules which bind to analyte, as described below. As used herein a "region" of a substrate or surface thereof refers to a contiguous portion of the substrate or surface thereof.

In an embodiment, the combination of an engineered surface and labeling nanoparticle has a detectable emission. In a further embodiment, the combination of an engineered surface and labeling nanoparticle has a detectable emission in the optical spectrum. In an embodiment, optical spectrum refers to light radiation that contains wavelengths from about 100 nm to about 1600 nm. In an embodiment, the combination of an engineered surface and labeling nanoparticle has a detectable emission in the visible spectrum. In an embodiment, visible spectrum refers to light radiation that contains wavelengths from about 360 nm to about 800 nm. In an embodiment, the combination of an engineered surface and labeling nanoparticle has a detectable emission in the ultraviolet spectrum. In an embodiment, ultraviolet spectrum refers to radiation with wavelengths less than that of visible light (i.e., less than approximately 360 nm) but greater than that of X-rays (i.e., greater than approximately 0.1 nm). In an embodiment, the combination of an engineered surface and labeling nanoparticle has a detectable emission in the infrared spectrum. In an embodiment, infrared spectrum refers to radiation with wavelengths of greater than 800 nm.

In an embodiment, the detectable emission is localized surface plasmon resonance. Localized surface plasmon resonance occurs when the correct wavelength of light strikes a metal nanostructure, causing the plasma of conduction electrons to oscillate collectively. This excitation causes selective photon absorption and generation of locally enhanced or amplified electromagnetic fields at the nanostructure surface. For example, the localized surface plasmon resonance for noble metal nanoparticles in the 20 to few hundred nanometer size regime occurs in the visible and IR regions of the spectrum and can be measured by UV-visible-IR extinction spectroscopy (Haynes et al., 2001, J Phys. Chem. B 105: 5599-5611).

In an embodiment the engineered surface includes materials and/or structures for interaction with the labeling nanoparticles. In an embodiment, the engineered surface includes high refractive index materials, such as but not limited to toTiO$_2$ and Al$_2$O$_3$, and Si. High refractive index materials refer to dielectric or insulating materials having bandgaps equal to or above 2.5 eV. In an embodiment, the engineered surface includes nanostructures such as, but not limited to nanoparticles, nanowires, nanotubes, and quantum dots. In a further embodiment, the nanostructures are formed from metal materials. In a still further embodiment, the nanostructures are formed from noble metals. In yet another embodiment, the nanostructures are formed from materials such as, but not limited to, copper (Cu), silver (Ag), gold (Au), or platinum (Pt). In an alternative embodiment, the nanostructures are formed from semiconducting materials, such as but not limited to CdTe, CdSe, ZnSe, ZnS, Si, Ge, and the class of semiconductors known as III-V made from (B, Al, Ga, In) and (N, P, As, Sb, and Bi) and combinations thereof.

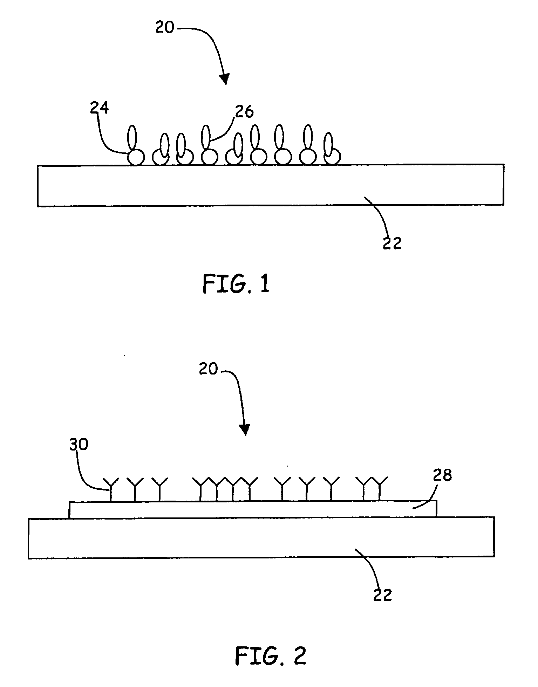

An embodiment of an engineered surface is schematically illustrated in Figure 1. Figure 1 illustrates a cross-section of one embodiment of an engineered surface. Engineered surface 20 is supported on substrate 22. Engineered surface 20 includes nanostructures 24. The engineered surface 20 additionally includes immobilized molecules 26 bound to nanostructures 24.

An alternative embodiment of an engineered surface is schematically illustrated in Figure 2. Figure 2 illustrates a cross-section of another embodiment of an engineered surface. Engineered surface 20 is supported on substrate 22. Engineered surface 20 includes layer 28. Immobilized molecules 30 are associated with layer 28 of engineered surface 20.

In an alternative embodiment, an engineered surface is formed on a tile less than 1000 $\mu$m (microns; 10$^{-6}$ m). Size of a tile refers generally to the dimensions of a region (i.e. length and width) of a surface for holding an engineered surface. In an embodiment, the surface dimensions of an individual tile are from about 5 $\mu$m to about 500 $\mu$m. In a further embodiment, the surface dimensions of an individual tile are from about 10 $\mu$m to about 100 $\mu$m. In an embodiment, each tile holds at least one engineered surface. In a further embodiment, each tile holds one engineered surface. In an embodiment, patterned tiles are applied to a substrate as a suspension or paste dispensed over the detection region.

In an embodiment, the combination of engineered surface and labeling nanoparticle is selected to increasing emission and/or narrowing the linewidth at a selected wavelength (or narrow band of wavelengths) of emission. In a further embodiment, an average desired combination of engineered surface and labeling nanoparticle is selected to improve the localized surface plasmon resonance at longer wavelengths. In a further embodiment, the detection is improved by selecting a resonance at a longer wavelength where the optical detector is more sensitive. In an embodiment, an optical detector is a Silicon photodiode. For example, Silicon photodiodes have better spectral responsivity from about 700nm to about 1000nm than outside of that range, e.g., 450nm.

In an embodiment, an engineered surface is formed on a region of a surface of a substrate by known techniques such as physical or chemical deposition, lithography, embossing, molding, or spotting. In an embodiment, the surface is prepared by known techniques such as lithography, embossing, molding, or spotting, and nanostructures are subsequently associated with the surface. In an embodiment, the surface of the support has modified surface chemistry for coupling nanostructures or immobilized molecules. In an embodiment, the modified surface chemistry includes, but is not limited to modifying the hydrophobicity or charge in each spot. In an embodiment, surface modification is accomplished by optical lithography, e-beam lithography, stamping or bottoms-up techniques such as nanosphere lithography, or assembly of block co-polymers.

In an embodiment, an engineered surface is formed by nanosphere lithography. Nanosphere lithography refers to a fabrication technique to produce engineered surfaces with precisely controlled shape, size, and interstructure spacing, and accordingly precisely controlled localized surface plasmon resonance. (Hulteen et al., 1195, J. Vac. Sci. Technol. A 13:1553-1558.) Structures built by nanosphere lithography begin with the self-assembly of size-monodispersed nanospheres to form a two-dimensional colloidal crystal deposition mask. Following self-assembly of the nanosphere mask, a noble metal or other material is then deposited by thermal evaporation, electron beam deposition, or pulsed laser deposition from a source normal to the substrate through the nanosphere mask to a controlled mass thickness, $d_m$. After noble metal deposition, the nanosphere mask is removed by sonicating the entire sample in a solvent, leaving behind the material deposited through the nanosphere mask to the substrate. The localized surface plasmon resonance of nanosphere lithography-derived structures depends on nanostructure material, size, shape, interstructure spacing, substrate, solvent, dielectric thin film overlayers, and molecular adsorbates. (Haynes et al., 2001, J. Phys. Chem. B., 105: 5599-5611)

In an embodiment, a substrate includes an array. An "array", unless a contrary intention appears, includes any one-, two- or three-dimensional arrangement of addressable regions bearing a particular chemical moiety or moieties (for example, biomolecules such as antibodies) associated with that region. In an embodiment, each region includes an engineered surface. An array is "addressable" in that it has multiple regions of different moieties (for example, different antibodies) such that a region (a "feature" or "spot" of the array) at a particular predetermined location (an "address") on the array will detect a particular target or class of targets (although a feature may incidentally detect non-targets of that feature). Array features are typically, but need not be, separated by intervening spaces. In the case of an array, the "analyte" will be referenced as a moiety in a mobile phase (typically fluid), to be detected by immobilized molecules which are bound to the substrate at the various regions. However, either of the "analyte" or "immobilized molecules" may be the one which is to be evaluated by the other. Immobilized molecules may be covalently bound to a surface of a non-porous or porous substrate either directly or through a linker molecule, or may be adsorbed to a surface using intermediate layers (such as polylysine) or porous substrates.

An "array layout" refers to one or more characteristics of the array or the features on it. Such characteristics include one or more of: feature positioning on the substrate; one or more feature dimensions; some indication of an identity or function (for example, chemical or biological) of a moiety at a given location; how the array should be handled (for example, conditions under which the array is exposed to a sample, or array reading specifications or controls following sample exposure).

**Engineered Surface and Immobilized Molecules**

In an embodiment, an engineered surface includes at least one immobilized molecule with binding affinity for an analyte. As used herein, the term "immobilized" is used with respect to molecules coupled to a support, region or array, and refers to molecules being stably oriented on the support, region, or array, so that they do not migrate. In an embodiment, immobilized molecules are coupled by covalent coupling, ionic interactions, electrostatic interactions, or van der Waals forces. In an embodiment, the immobilized molecules are coupled to an engineered surface. In a further embodiment, the immobilized molecules are coupled to the nanostructures. In another further embodiment, the immobilized molecules are coupled to the substrate surface. In a still further embodiment, the immobilized molecules are coupled to the substrate surface between the nanostructures.

In a further embodiment, an immobilized molecule has specific binding affinity for an analyte. Binding affinity refers to how tight an analyte or other ligand binds to an immobilized molecule. Mathematically, affinity is $1/K_d$, wherein $K_d$ is $k_2/k_1$ where $k_2$ is the rate of dissociation of the analyte from the immobilized molecule and $k_1$ is the rate of association of the analyte for the immobilized molecule. The higher the affinity (lower the $K_d$) the tight the analyte binds to the immobilized molecule. Specific binding affinity refers to the ability of the immobilized molecule to discriminate among competing ligands. In a further embodiment, specific binding affinity refers to the ability of the immobilized molecule to preferentially bind a analyte when other competing ligands are present in a sample.

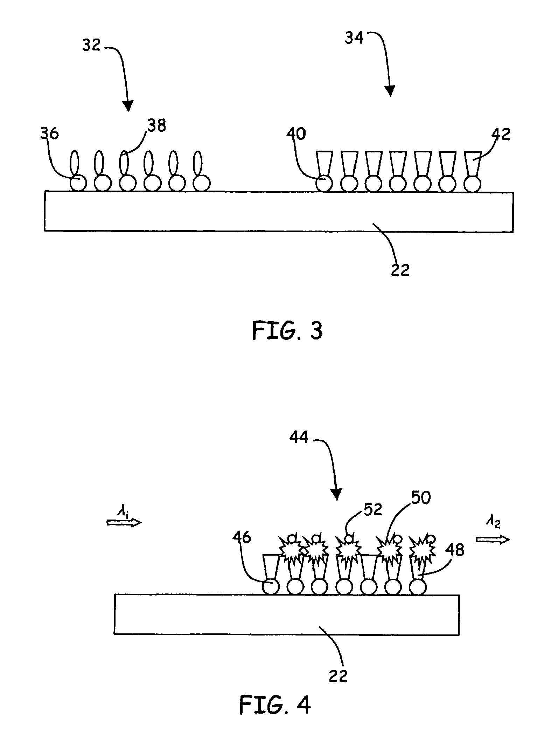

In an embodiment, a substrate for use in POCT includes two or more engineered surfaces. An embodiment of a substrate including two or more engineered surfaces is illustrated in Figure 3. Figure 3 illustrates cross-sections of one embodiment of a substrate 22 including a first engineered surface 32 and a second engineered surface 34. First engineered surface 32 includes a plurality of nanostructure 36 and immobilized molecule 38. Second engineered surface 34 includes a plurality of nanostructure 40 and immobilized molecule 42. In an embodiment, immobilized molecules 38 and 42 are different molecules.

In an embodiment, an engineered surface includes a plurality of immobilized molecules of one chemical identity. In embodiments, where a substrate includes two or more engineered surfaces, each engineered surface includes the same immobilized molecule. In an embodiment, a substrate comprises a first array including a first plurality of immobilized molecules, and a second array including a second plurality of immobilized molecules, wherein the first plurality and second plurality comprise the immobilized molecules of common identity. In an embodiment, a substrate includes two or more engineered surfaces, each engineered surface includes immobilized molecules with different identity between the arrays. In an embodiment, a substrate includes a first engineered surface including first immobilized molecules, and a second engineered surface including second immobilized molecules, wherein the first immobilized molecules are different from the second immobilized molecules.

In an embodiment, an immobilized molecule binds at least one analyte molecule. In an embodiment, an immobilized molecule binds at least one analyte with sufficient affinity for detection of the interaction, as described below. In an embodiment, an immobilized molecule has specific binding affinity for an analyte. In an embodiment, the immobilized molecule is selected for inclusion in an engineered surface for its ability to bind the analyte molecule.

The term "analyte" refers to any molecule or atom or molecular complex suitable for detection. In an embodiment, example analytes include, but are not limited to, various biomolecules (e.g., proteins, nucleic acids, lipids, etc.), glucose, ascorbate, lactic acid, urea, pesticides, chemical warfare agents, pollutants, and explosives. In an embodiment, an analyte is one component of a sample, which likely contains multiple ligands. In an embodiment, the analyte molecule is the substance to be detected which may be present in a sample for testing in POCT.

In an embodiment, at least one engineered surface includes immobilized molecules having non-specific binding properties. Non-specific binding refers to molecules that bind indiscriminately to analyte and/or additional ligands present in a sample. In an embodiment, immobilized molecules with non-specific binding affinity bind multiple ligands from a sample. In an embodiment, an immobilized molecule has non-specific binding affinity for an analyte and other ligands. In an embodiment, immobilized molecules with non-specific binding affinity to bind multiple ligands for use as a control are compared to immobilized molecules with specific binding affinity for the analyte being detected.

In an embodiment, an engineered surface includes immobilized molecules with non-specific binding properties for use as a reference channel or control for measurement of analyte binding in other arrays. In an embodiment, a substrate includes a control engineered surface and one or more additional engineered surfaces. In an embodiment, a substrate includes a first array and a second array, wherein the second array is a control array. In a further embodiment, an engineered surface for use as a reference channel or control is formed of the same nanostructure materials and in the same manner as one or more other arrays on the substrate.

In an embodiment, an engineered surface includes immobilized molecules with non-specific binding properties for use as a positive control. In an embodiment, a positive control has immobilized molecules bound with nanoparticle-labeled compounds. In a further embodiment, detection of an engineered surface is a positive control gives 100% response. In an embodiment, a positive control is used as a reference channel for measurement of analyte binding in other channels, regions or arrays.

In an embodiment, an immobilized molecule and an analyte are any binding pair including, but not limited to, antibody/antigen, antibody/antibody, antibody/antibody fragment, antibody/antibody receptor, antibody/protein A or protein G, hapten/anti-hapten, biotin/avidin, biotin/streptavidin, folic acid/folate binding protein, vitamin B 12/intrinsic factor, nucleic acid/complementary nucleic acid (e.g., DNA, RNA, PNA), and chemical reactive group/complementary chemical reactive group (e.g., sulfhydryl/maleimide, sulfhydryl/haloacetyl derivative, amine/isotriocyanate, amine/succinimidyl ester, and amine/sulfonyl halides).

In certain embodiments the immobilized molecule is a cellular binding partner of the analyte. For example, where the analyte is a subregion of a receptor protein kinase such as EGF receptor, the binding partner is EGF or a functional fragment thereof; where the analyte is a nucleic acid, the binding partner sometimes is a transcription factor or histone or a functional portion thereof; or where the analyte is a glycosyl moiety, the binding partner sometimes is a glycosyl binding protein or a portion thereof.

The analyte can include a protein, a peptide, an amino acid, a hormone, a steroid, a vitamin, a drug including those administered for therapeutic purposes as well as those administered for illicit purposes, a bacterium, a virus, and metabolites of or antibodies to any of the above substances. In particular, such analytes include, but are not intended to be limited to, ferritin; creatinine kinase MB (CK-MB); digoxin; phenytoin; phenobarbital; carbamazepine; vancomycin; gentamicin, theophylline; valproic acid; quinidine; luteinizing hormone (LH); follicle stimulating hormone (FSH); estradiol, progesterone; IgE antibodies; vitamin B2 micro-globulin; glycated hemoglobin (Gly Hb); cortisol; digitoxin; N-acetylprocainamide (NAPA); procainamide; antibodies to rubella, such as rubella-IgG and rubella-IgM; antibodies to toxoplasma, such as toxoplasmosis IgG (Toxo-IgG) and toxoplasmosis IgM (Toxo-IgM); testosterone; salicylates; acetaminophen; hepatitis B core antigen, such as anti-hepatitis B core antigen IgG and IgM (Anti-HBC); human immune deficiency virus 1 and 2 (HIV 1 and 2); human T-cell leukemia virus 1 and 2 (HTLV); hepatitis B antigen (HBAg); antibodies to hepatitis B antigen (Anti-HB); thyroid stimulating hormone (TSH); thyroxine (T4); total triiodothyronine (Total T3); free triiodothyronine (Free T3); carcinoembryonic antigen (CEA); and alpha fetal protein (AFP). Drugs of abuse and controlled substances include, but are not intended to be limited to, amphetamine; methamphetamine; barbiturates such as amobarbital, secobarbital, pentobarbital, phenobarbital, and barbital; benzodiazepines such as librium and valium; cannabinoids such as hashish and marijuana; cocaine; fentanyl; LSD; methaqualone; opiates such as heroin, morphine, codeine, hydromorphone, hydrocodone, methadone, oxycodone, oxymorphone, and opium; phencyclidine; and propoxyphene. The details for the preparation of such antibodies and their suitability for use as specific binding members are well known to those skilled in the art.

In an embodiment, the immobilized molecules are an antibodies having binding affinity for the analyte. In a further embodiment, an engineered surface includes randomly distributed antibodies. In a further embodiment, the engineered surface is patterned or chemically modified for binding antibodies.

**Labeling Nanoparticles**

In an embodiment, labeling nanoparticles are bound to the analyte to be detected. In an embodiment, labeling nanoparticles are bound to many ligands (e.g., sample components), including the analyte (if present) in a sample. Labeling nanoparticles are bound to analyte and other ligands in a sample by known techniques. In an embodiment, labeling nanoparticles are bound to analyte by cross-linking chemistry. In an embodiment, cross-linking agents are selected based on reactivity with the labeling nanoparticle and analyte.

In a further embodiment, an EDC (1-ethyl-3-(3-dimethylamino propyl) carbodiimide hydrochloride)/sulfo-NHS (N-hydroxy-sulfosuccinimide) cross-linking procedure is used. For example, a sample is mixed with freshly prepared solutions of 0.2M EDC and 25mM NHS, followed by addition of labeling nanoparticles. In an alternative example, gold labeling nanoparticles are prepared by mixing with $HS(CH_2)_2CH_3$ and $HS(CH_2)_2COOH$ for 24 hours with stirring and subsequently reacting with ECD and NHS for 30 minutes. This preparation is subsequently added to a sample or analyte to be labeled.

In an embodiment, labeling nanoparticles refer to solid metal particles of nanoscale size. In an embodiment, labeling nanoparticles are solid metal particles of a metal for which surface plasmons are excited by visible radiation. In a further embodiment, labeling nanoparticles are solid metal particles of copper (Cu), silver (Ag), gold (Au) or platinum (Pt).

In an embodiment, labeling nanoparticles refer to nanoscale size cores (e.g., nanospheres) coated with metal layers (e.g., metal nanoshells). In an embodiment, the core diameter and the metal thickness of nanoshells can be varied to modify the SERS properties of the nanoparticles. See, for example, R. L. Moody, T. Vo-Dinh, and W. H. Fletcher, "Investigation of Experimental Parameters for Surface-Enhanced Raman Spectroscopy," Appl. Spectrosc., 41, 966 (1987). In an embodiment, nanospheres are formed of dielectric materials. In an embodiment, nanospheres are coated with a thin layer of metal for which surface plasmons are excited by visible radiation. In a further embodiment, the nanoshells are formed from metal of copper (Cu), silver (Ag), or gold (Au). In an embodiment, nanospheres are coated with a thin layer of metal for which surface plasmons are excited by visible radiation. In a further embodiment, the nanoshells are formed from noble metals, such as of copper (Cu), silver (Ag), gold (Au), or platinum (Pt).

In an embodiment, labeling nanoparticles have an average diameter from about 1nm to about 1000nm. In a still further embodiment, nanoparticles have an average diameter from about 10nm to about 500 nm. In a yet another embodiment, labeling nanoparticles have an average diameter from about 10 nm to about 100 nm. In a final embodiment, labeling nanoparticles have an average diameter from about 10 nm to about 50 nm.

In an embodiment, labeling nanoparticles are about the same size as the array nanostructures. In an embodiment, the labeling nanoparticles are different in size from the array nanostructures. In an embodiment, the labeling nanoparticles are formed from the same or similar material to the array nanostructures. In an embodiment, the labeling nanoparticles are formed from a different material from the array nanostructures.

**Methods**

In an embodiment, POCT devices utilize substrates including engineered surfaces for use in assays between immobilized affinity molecules and analyte molecules to detect the presence of particular analytes in a sample. The immobilized affinity molecules are molecules capable of binding with target analytes.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Environmental samples include environmental material such as surface matter, soil, water, crystals and industrial samples. Such examples are not however to be construed as limiting the sample types applicable to the present invention.

As used herein, a device configured for the detection of signal from a nanoparticle array refers to any device suitable for detection of a signal from an engineered surface and/or nanoparticles associated with an engineered surface. In some embodiments, the device includes delivery and collection optics, a laser or LED source, a notch filter, and detector. In an embodiment, the device includes optical readers. In an embodiment, the eye of an optical reader detects light scattered by the engineered surface and/or associated nanoparticles by their surface plasmon resonance.

An embodiment of a substrate for use in an assay is shown in Figure 4. Figure 4, illustrates a sectional view of one embodiment of a substrate 22 including an engineered surface 44. Engineered surface 44 includes a plurality of nanostructure 46 and immobilized molecule 48. Analyte 50 is labeled with labeling nanoparticle 52. Additionally, analyte 50 is bound to immobilized molecule 48. Binding interaction of analyte 50 to immobilized molecule 48 brings labeling nanoparticles 50 in close proximity to engineered surface 44. The binding of analyte 50 is detected by optical spectroscopy at least one emission peak, $\lambda_2$, following irradiation of the engineered surface 44 with incident light $\lambda_i$.

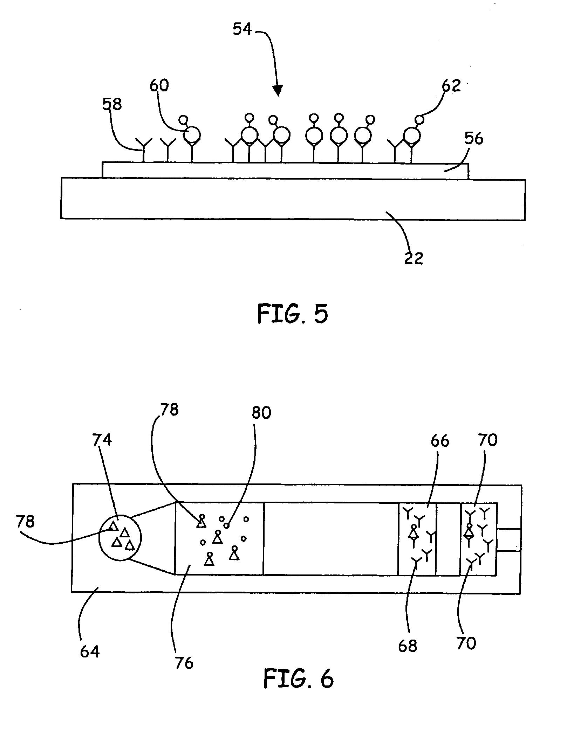

An additional embodiment of a substrate for use in an assay is shown in Figure 5. In the embodiment of Figure 5, substrate 22 includes engineered surface 54, wherein engineered surface 54 has a layer of high refractive index material 56. Immobilized molecules 58 are associated with the layer of high refractive index material 56. Analytes 60 with labeling nanoparticles 62 are shown associated with the immobilized molecules 58. Binding interaction of analytes 60 to immobilized molecules 58 brings labeling nanoparticles 62 in close proximity to engineered surface 54. As further example embodiment, immobilized molecules 58 are illustrated as antibodies. In a further embodiment, nanoparticles 62 are formed of metal materials.

A further embodiment is illustrated in Figure 6. Figure 6 shows a cross-sectional view of one embodiment of a substrate 64 suitable for use in POCT. In an embodiment, substrate 64 includes a first engineered surface 66 with first immobilized molecule 68 and a second engineered surface 70 with second immobilized molecule 72. Substrate 64 additionally comprises a sample opening 74 and labeling area 76. In an embodiment, a sample containing analyte 78 is placed on substrate 64 at sample opening 74. Sample opening 74 is connected to labeling area 76, such that the sample with analyte 78 flows into labeling area 76. In labeling area 76, the sample with analyte 78 is labeled with nanoparticles 80. Labeling area 76 is connected with first engineered surface 66 and second engineered surface 70, such that the sample with analyte 78 flows into those areas. In an embodiment, first engineered surface 66 has associated immobilized molecules 68 with specific binding affinity for analyte 78. In an embodiment, second engineered surface 70 has associated immobilized molecules 72 with non-specific binding affinity for analyte 78. In an embodiment, analyte 78 and/or other sample components (not shown) that do not bind to the immobilized molecules are removed from the substrate (e.g., by washing) before detection. In an embodiment, substrate 64 is exposed to a source (not shown) and emission from the combination of labeling nanoparticles associated with one or both engineered surfaces is detected.

Many types of assays can be carried out with a substrate including at least one engineered surface for a wide variety of analytes labeled with nanoparticles. In an embodiment, assays that can be performed include, but are not limited to, general chemistry assays and immunoassays. In an embodiment, qualitative assays are performed. In an embodiment, quantitative assays are performed.

In an embodiment, a single assay is performed. In a further embodiment, a substrate for performing a single assay includes a single engineered surface. In another embodiment, a substrate for performing a single assay includes multiple engineered surfaces.

In an embodiment multiple assays can be done at one time. In a further embodiment, a substrate includes multiple engineered surfaces. In a still further embodiment, a substrate includes multiple engineered surfaces that are non-identical. Non-identical engineered surface refers to differences between two or more arrays, including but not limited to: nanostructure material, particle size or shape, interstructure spacing, and identity of immobilized molecule. For example, in an embodiment, a substrate includes an array for detecting total cholesterol and another array for detecting HDL cholesterol from a single sample. In various embodiments, a substrate includes various numbers of engineered surfaces to analyze to measure one, two, three, or more analytes at one time.

One typical array assay method involves a substrate including at least one engineered surface, wherein at least one engineered surface includes an immobilized molecule, wherein the immobilized molecule has specific binding affinity for an analyte. Each analyte is associated with at least one labeling nanoparticle. A solution containing the nanoparticle-labeled analytes is placed in contact with the substrate in the region including the engineered surface under conditions sufficient to promote binding of analytes in the solution to the array. In an embodiment, the assay method includes a washing step to remove unbound sample. Binding of the analytes to the immobilized molecules forms a binding complex that is bound to the engineered surface and includes at least one labeling nanoparticle.

In an embodiment, the proximity of the nanoparticle-labeled analyte to the engineered surface creates a combination. In a further embodiment, the combination is described as a hybrid self-assembled superstructure. In an embodiment, the combination of engineered surface and labeling nanoparticles gives a different emission spectra than a substrate including labeling nanoparticles but lacking an engineered surface. The binding of at least one nanoparticle-labeled analyte to at least one affinity molecule causes a detectable emission upon excitiation of the combination of the engineered surface and labeling nanoparticle with a source (e.g., for optical spectroscopy). In an embodiment, a detectable emission or resonance of an engineered surface is attributable to the emission or resonance of the nanostructures. In an embodiment, a detectable emission or resonance of an engineered surface is attributable to the emission or resonance of at least one nanoparticle-labeled analyte associated with the engineered surface. In an embodiment, a detectable emission or resonance of an engineered surface is attributable to the emission or resonance of the nanostructures and the emission or resonance of at least one nanoparticle-labeled analyte associated with the engineered surface.

In an embodiment, the detected emission or resonance wavelength of bound nanoparticle-labeled molecules is $\lambda_1$. In an embodiment, there is a detectable difference in emission between when labeling nanoparticles are associated to the engineered surface and when labeling nanoparticles are not associated to the engineered surface. In a further embodiment, the detectable difference is a shift in detected wavelength, e.g., $\lambda_1 \to \lambda_2$. In an alternative embodiment, the detectable difference is an increase or decrease in emission strength (e.g., intensity). In a further embodiment, the difference in emission intensity is at $\lambda_1$, wherein the source is $\lambda_i$.

In an embodiment, the engineered surface is excited by a source for optical spectroscopy. In an embodiment, the source includes wide band sources or narrow band sources, such as lasers. In an embodiment, source excitation of the engineered surface causes plasmon resonance detected by a suitable detector. In an embodiment, the emission or resonance or difference therein is detectable with the human eye. In an embodiment, the emission pattern of an engineered surface is digitally scanned for computer analysis. In various embodiments, engineered surface emission spectra can be used to generate data for chemical analysis. In further various embodiments, data is used for, but not limited to, the identification of drug ligands, single-nucleotide polymorphism mapping, monitoring samples from patients to track their response to treatment, and assessing the efficacy of new treatments.

The sample to be tested for the presence of an analyte can be derived from any biological source, such as a physiological fluid, including whole blood or whole blood components including red blood cells, white blood cells, platelets, serum and plasma; ascites; urine; sweat; milk; synovial fluid; peritoneal fluid; amniotic fluid; cerebrospinal fluid; and other constituents of the body which may contain the analyte of interest. The test sample can be pre-treated prior to use, such as suspension or dilution in an solution, e.g., aqueous buffer solution, preparing plasma from blood, diluting viscous fluids, or the like; methods of treatment can involve filtration, distillation, concentration, and the addition of reagents. Besides physiological fluids, other liquid samples can be used such as water, food products and the like for the performance of environmental or food production assays. In addition, a solid material suspected of containing the analyte can be used as the test sample. In some instances it may be beneficial to modify a solid test sample to form a liquid medium or to release the analyte. The analyte can be any compound or composition to be detected or measured and which has at least one epitope or binding site.

**Demonstrative Examples**

Example 1

In a first example embodiment, an engineered surface for detection of an analyte in a sample includes an array of noble metal nanoparticles in a designated pattern having localized surface plasmon resonance. In an embodiment, immobilized molecules coupled to the array are antibodies with binding affinity for the analyte. In an embodiment, the nanoparticles are gold or silver nanoparticles. The pattern of nanoparticles in the engineered surface is selected such that excitation of the array (with no bound analyte) with source at an initial wavelength $\lambda i$, causes the array to resonate at a given wavelength $\lambda_1$. In an embodiment, $\lambda_1$ is a wavelength in the visible range that gives the array a characteristic color.

Binding of analyte or other ligand tagged with gold or silver nanoparticles to the engineered surface through antibody-antigen recognition chemistry, causes perturbation or disruption of the resonant structure of the engineered surface. This leads to a dramatic shift in the color scattering upon excitation at initial wavelength $\lambda i$ (e.g., $\lambda i \to \lambda_2$, wherein $\lambda_1 \neq \lambda_2$). Therefore, in an embodiment, binding of analyte to the engineered surface is measured by a comparison of detected emission or resonant wavelength to either or both $\lambda_2$ (nanoparticle-labeled analyte bound) or $\lambda_1$ (nanoparticle-labeled analyte not bound). In a further embodiment, either or both $\lambda_2$ (nanoparticle-labeled analyte bound) or $\lambda_1$ (nanoparticle-labeled analyte not bound) is color in the visible spectrum. In a further embodiment, the color at $\lambda_1$ and/or the color at $\lambda_2$ are detectable by the human eye.

Example 2

In a second example embodiment, an engineered surface for detection of an analyte in a sample includes an array of high refractive index material. In an embodiment, the array includes nanostructures of $TiO_2$. In a further embodiment, the substrate is also a high refractive index material, such as $TiO_2$. As described above, analyte or other ligands labeled with noble metal nanoparticles bind to the engineered surface by affinity binding to one or more immobilized molecules. The surface plasmon resonance of the nanoparticles shifts as a function of the surrounding dielectric medium. In the "Rayleigh Approximation," where the particle is much smaller than the wavelength of light, the resonance condition is

$$\varepsilon(\omega)_m = \varepsilon_d \left(1 - 1/A\right) \qquad \text{Equation 1}$$

where $\varepsilon(\omega)_m$ is the dielectric constant of the metal and $\varepsilon_d$ is the dielectric constant of the surrounding dielectric, and A is the depolarization factor. Consequently, proximity of the nanoparticle-bound analyte to the array changes $\varepsilon_d$ for the nanoparticle, thereby changing the wavelength at which the particle resonates. Therefore, in an embodiment, binding of analyte to the engineered surface changes the surface plasmon resonant wavelength of nanoparticles associated with the array.

Example 3

In a third example embodiment, an engineered surface for detection of an analyte in a sample includes an engineered surface including quantum dots. In an embodiment, quantum dots, characterized by bandgap energy of hc/$\lambda_1$, are attached to the substrate. In an embodiment, excitation of the array (with no bound analyte) with source at an initial wavelength $\lambda i$, causes the array to resonate at a given wavelength $\lambda_1$. In an embodiment, the quantum dots are attached to the substrate by a linker moiety.

As described above, analyte or other ligands labeled with noble metal nanoparticles bind to the engineered surface by affinity binding to one or more immobilized molecules. In an embodiment, immobilized molecules coupled to the array are antibodies with binding affinity for the analyte.

When the metal nanoparticle-coupled analyte binds to the immobilized molecule, the quantum dots and the metal nanoparticles are brought into close proximity. Consequently, the emission characteristics of the quantum dots shift. For further explanation of theory see, B. Nikoobakht et al., 2002, Photochemistry and Photobiology 75:591. With close proximity of a few nanometers to a metal nanoparticle, the fluorescence of a quantum dot is quenched severely by the increase in non-radiative pathways through the metal nanoparticle. Nanoparticle-coupled analyte binding to the engineered surface is measured by quantitating the change (i.e. decrease) in emission intensity of the quantum dots in the array. In an embodiment, the change in emission intensity is at $\lambda_1$.

In an alternative embodiment to example 3, the nanoparticles and the quantum dots are exchanged. The engineered surface includes the nanoparticles, while the quantum dots are coupled to the analyte. Measurement is performed in substantially the same manner.

Example 4

In a third example embodiment, an engineered surface for detection of an analyte in a sample includes an engineered surface including quantum dots. In an embodiment, quantum dots, characterized by bandgap energy of hc/$\lambda_1$, are attached to the substrate. As described above, analyte or other ligands labeled with noble metal nanoparticles bind to the engineered surface by affinity binding to one or more immobilized molecules. In an embodiment, immobilized molecules coupled to the array are antibodies with binding affinity for the analyte. When the nanoparticle-coupled analyte binds to the immobilized molecule, the array of quantum dots and nanoparticle are brought into close proximity.

The close proximity of at least one quantum dot and at least one nanoparticle provide surface plasmon amplification by stimulated emission of radiation. This is alternatively described as forming a "SPASER". See, Bergman and Stockman, 2002, Physics Research Letters, 027402-1-4. In this example embodiment, a quantum dot is excited (e.g., by a radiation or light source such as a laser) and transfers a least a portion of the energy by radiationless energy transfer to the nanoparticles. The radiationless energy transfer excites localized surface plasmon modes in the metal nanoparticle. The metal nanoparticle acts as a resonator, thereby building up a large number of surface plasmons in a single mode. In an embodiment, the detected mode resonates at a different wavelength from the quantum dot.

In an embodiment, the metal nanoparticle is not resonant by itself at the wavelength of light ($\lambda_i$) incident on the quantum dot. In a further embodiment, the quantum dot has a high absorption cross-section at $\lambda_i$. When the quantum dot and metal nanoparticle are in close proximity through affinity binding of immobilized molecule to analyte, the quantum dot transfers energy into surface plasmons with energy hc/$\lambda_2$ in the metal nanoparticle. Detection of localized surface plasmon resonance is performed by measuring the emission (signal) at wavelength $\lambda_2$.

All publications and patent applications in this specification are indicative of the level of ordinary skill in the art to which this invention pertains and are incorporated herein by reference in their entireties.

The various embodiments described above are provided by way of illustration only and should not be construed to limit the invention. Those skilled in the art will readily recognize various modifications and changes that may be made to the present invention without following the example embodiments and applications illustrated and described herein, and without departing from the true scope of the present invention, which is set forth in the following claims.

**Claims**

1. A substrate (22) for use in detection of an analyte in point of care testing of a sample, the substrate comprising:

   an engineered surface (32) on the substrate;
   a first molecule (38) with specific binding affinity for the analyte associated with the engineered surface;

   wherein at least one labeling nanoparticle (36) is associated with the analyte, and at least one labeling nanoparticle interacts with the engineered surface when analyte binds to a first molecule; and
   wherein the interaction of the labeling nanoparticle with the engineered surface has a detectable resonance.

2. The substrate of claim 1, wherein the engineered surface comprises a plurality of nanostructures, wherein the nanostructures are selected from a group consisting of nanowires, nanotubes, nanoparticles, or combinations thereof.

3. The substrate of claims 1-2, wherein the engineered surface comprises high refractive index materials.

4. The substrate of claims 1-4, wherein the labeling nanoparticle is selected from the group consisting of: metal nanoparticles, semiconducting nanoparticles, nanowires, and nanotubes.

5. The substrate of claims 1-4, wherein the substrate additionally comprises a second molecule (42) with non-specific binding affinity for the analyte associated with a second engineered surface (34); and wherein at least one labeling nanoparticle (40) interacts with the second engineered surface when analyte binds to the second molecule.

6. A kit for use in detection of an analyte from a sample, the kit comprising:

   a substrate of any one of claims 1-5; and
   a composition comprising at least one labeling nanoparticle, wherein the composition is combinable with the sample so as to label the analyte with at least one nanoparticle;

   wherein the binding of at least one nanoparticle-labeled analyte to at least one affinity molecule causes a detectable resonance, $\lambda_1$.

7. The kit of claim 6, wherein the detectable resonance is a wavelength $\lambda_1$ from the optical range.

8. The kit of claim 6, wherein the the detectable resonance is visibly detectable with a characteristic color.

9. The kit of claim 6, wherein the detectable resonance in $\lambda_1$ is a change in emission intensity at $\lambda_1$, or is the localized surface plasmon resonance of labeling nanoparticle in proximity to the engineered surface.

10. A method of detecting or identifying an analyte in a sample by spectroscopy comprising:

    binding labeling nanoparticles to analyte in the sample;
    exposing the sample to a substrate (22) comprising:

    a first engineered surface (32) having at least one molecule (38) associated therewith, the molecule having specific binding affinity for the analyte;
    a second engineered surface (34) having at least one immobilized molecule (42) is associated therewith, the molecule having non-specific binding affinity for the analyte;

    irradiating the first engineered surface with an excitation source;
    detecting emission wavelength or intensity of the associated first engineered surface with any nanoparticle-bound-analyte;
    irradiating the second engineered surface with an excitation source;
    detecting resonance of the associated second engineered surface with any nanoparticle-bound-analyte;
    comparing the binding of analyte to the first engineered surface to the binding of analyte to the second engineered surface by comparing the detected resonances.

20

24          26

22

FIG. 1

20

30                          28

22

FIG. 2

FIG. 3

FIG. 4

FIG. 5

FIG. 6

**European Patent Office**

**EUROPEAN SEARCH REPORT**

Application Number

EP 06 25 3574

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/042403 A2 (LUDWIG MAXIMILIAN UNIVERSITAE [DE]; ROCHE DIAGNOSTICS GMBH [DE]; FELD) 21 May 2004 (2004-05-21) * figure 11 * * page 18, line 18 - page 19, line 1 * * page 5, line 36 - page 7, line 14 * * page 8, line 1 - page 10, line 11 * | 1-10 | INV. G01N33/542 ADD. G01N33/543 |
| X | WO 01/35081 A (PENN STATE RES FOUND [US]; NATAN MICHAEL J [US]) 17 May 2001 (2001-05-17) * page 3, line 7 - line 14 * * page 4, line 4 - line 10 * * page 4, line 20 - page 5, line 17 * * page 9, line 29 - line 31 * * page 14, line 27 - line 31 * * page 17, line 23 - line 31 * * figures 6,12 * * examples 2,3 * | 1-10 | |
| X | WO 01/09388 A (PENN STATE RES FOUND [US]; NATAN MICHAEL J [US]; GOODRICH GLENN [US];) 8 February 2001 (2001-02-08) * page 3, line 25 - page 5, line 31 * * page 5, line 6 - line 19 * * page 7, line 26 - line 31 * * page 12, line 11 - page 13, line 13 * * figures 6,12 * * examples 2,3 * | 1-10 | TECHNICAL FIELDS SEARCHED (IPC) G01N |
| A | WO 03/050291 A2 (UNIV DUKE [US]; CHILKOTI ASHUTOSH [US]; NATH NIDHI [US]; FREY WOLFGANG) 19 June 2003 (2003-06-19) * claims 1-20 * * figures 1,2 * * page 8, line 15 - line 21 * * page 10, line 4 - line 11 * | 1-10 | |

-/--

The present search report has been drawn up for all claims

| Place of search | Date of completion of the search | Examiner |
|---|---|---|
| The Hague | 27 October 2006 | VAN DER KOOIJ, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 1 744 161 A1

**European Patent Office**

## EUROPEAN SEARCH REPORT

Application Number

EP 06 25 3574

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2005/136532 A1 (LIN PAO-HUNG [TW]) 23 June 2005 (2005-06-23) * claims 8-10,12 * ----- | 1-10 | |

TECHNICAL FIELDS SEARCHED (IPC)

The present search report has been drawn up for all claims

| Place of search | Date of completion of the search | Examiner |
|---|---|---|
| The Hague | 27 October 2006 | VAN DER KOOIJ, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

15

## ANNEX TO THE EUROPEAN SEARCH REPORT
## ON EUROPEAN PATENT APPLICATION NO.

EP 06 25 3574

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2006

| Patent document cited in search report | | Publication date | Patent family member(s) | | Publication date |
|---|---|---|---|---|---|
| WO 2004042403 | A2 | 21-05-2004 | AU | 2003289853 A1 | 07-06-2004 |
| WO 0135081 | A | 17-05-2001 | AU | 3081301 A | 06-06-2001 |
| | | | CA | 2391009 A1 | 17-05-2001 |
| | | | EP | 1236034 A1 | 04-09-2002 |
| | | | JP | 2003514224 T | 15-04-2003 |
| WO 0109388 | A | 08-02-2001 | AU | 6504900 A | 19-02-2001 |
| | | | CA | 2381568 A1 | 08-02-2001 |
| | | | EP | 1212458 A1 | 12-06-2002 |
| WO 03050291 | A2 | 19-06-2003 | AU | 2002364120 A1 | 23-06-2003 |
| | | | US | 2003170687 A1 | 11-09-2003 |
| US 2005136532 | A1 | 23-06-2005 | NONE | | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HAYNES et al.** *J Phys. Chem. B,* 2001, vol. 105, 5599-5611
- **HULTEEN et al.** *J. Vac. Sci. Technol. A,* vol. 13, 1553-1558
- **HAYNES et al.** *J. Phys. Chem. B.,* 2001, vol. 105, 5599-5611

- **R. L. MOODY ; T. VO-DINH ; W. H. FLETCHER.** Investigation of Experimental Parameters for Surface-Enhanced Raman Spectroscopy. *Appl. Spectrosc.,* 1987, vol. 41, 966
- **B. NIKOOBAKHT et al.** *Photochemistry and Photobiology,* 2002, vol. 75, 591
- **BERGMAN ; STOCKMAN.** *Physics Research Letters,* 2002